# EUROPEAN PATENT SPECIFICATION

(11) **EP 2 939 161 B1**
(45) Date of publication and mention of the grant of the patent: **25.07.2018**
(21) Application number: 13834374.4
(22) Date of filing: 26.12.2013
(51) Int. Cl.: G06F 19/00

(54) **MONITOR DEFIBRILLATOR TELEMEDICINE SERVER**
TELEMEDIZINISCHER SERVER ZUR DEFIBRILLATORÜBERWACHUNG
MONITEUR, DÉFIBRILLATEUR ET SERVEUR DE TÉLÉMÉDECINE

(30) Priority: 26.12.2012 US 201261745833 P
(43) Date of publication of application: 04.11.2015
(73) Proprietor: Koninklijke Philips N.V., 5656 AE Eindhoven (NL)
(72) Inventor: GUINEY, Patrick, 5656 AE Eindhoven (NL); ILLIKKAL, Mashood Puthan Beetil, 5656 AE Eindhoven (NL); GRUBE, William Douglas, 5656 AE Eindhoven (NL); LANDER, Todd, 5656 AE Eindhoven (NL)
(74) Representative: Coops, Peter
(86) International application number: PCT/IB2013/061334
(87) International publication number: WO 2014/102726

(56) References cited:
- WO-A2-2006/071711
- US-A1- 2006 030 904
- US-A1- 2010 250 697
- STEINFELD E F: "INTERNET-APPLIANCE TECHNOLOGY AUTOMATES TEST EQUIPMENT", EDN ELECTRICAL DESIGN NEWS.(TEXAS INSTRUMENT), REED BUSINESS INFORMATION, HIGHLANDS RANCH, CO, US, vol. 45, no. 21, 12 October 2000 (2000-10-12), XP001143050, ISSN: 0012-7515
- RICK MERRIT: "Wireless Hospital, Health Care Products On The Upswing", INTERNET CITATION, 7 January 2004 (2004-01-07), XP002348225, Retrieved from the Internet: URL:http://www.techweb.com/article/printab leArticleSrc.jhtml?articleID=26803705 [retrieved on 2005-10-07]
- STEINFELD E F ET AL: "Is Embedded Going Net-Crazy??A Response", INTERNET CITATION, 29 March 2001 (2001-03-29), XP002348224, Retrieved from the Internet: URL:http://www.developonline.com/community /ed_resource/feature_article/13699?print [retrieved on 2005-10-05]

## Description

The invention relates to an improved apparatus and method for obtaining patient data from a monitor defibrillator for use at a remote station. The monitor defibrillator is integrated with a telemedicine server, such that external systems may use a standard communications interface and a read-only communications protocol to obtain the data.

Monitor defibrillators collect clinical data from a wide spectrum of patients that span the range from healthy to critically ill. Many such monitor defibrillators are employed by emergency medical system (EMS) caregivers at locations away from healthcare facilities. It is widely acknowledged that patient outcomes are improved when the clinical data collected by EMS is transmitted to the healthcare facility before the arrival of the patient.

Transmission of clinical data from monitor defibrillators to healthcare facilities serves multiple purposes. The transmission alerts the care facility to the clinical condition of the incoming patient. Sharing of the data allows consultation between the field medic and the caregiver at the care facility. Also, routine transmission of clinical data to a central location enables the assembly and archival storage of electronic patient care records (ePCRs).

Recent healthcare reform efforts have also prompted a greater emphasis on the need for EMS caregivers to manage and transmit the data associated with EMS patient care. Accurate and comprehensive patient care records are acknowledged to be a prerequisite for improved accountability of quality care, more efficient billing procedures, and reduced litigation that results from medical responses that have negative outcomes.

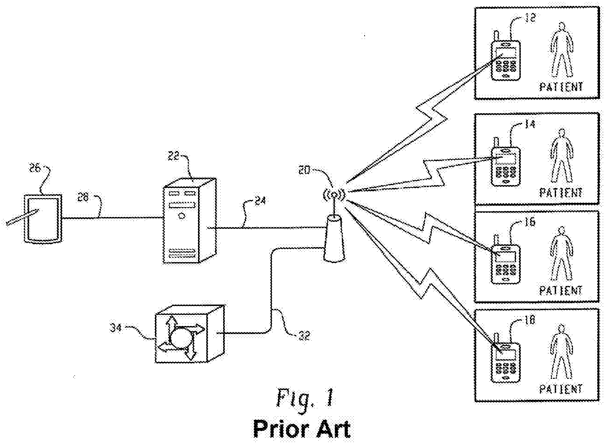

The necessity to transmit patient data from the patient's location to a central location at the hospital has given rise to a number of medical devices having integrated telemetry circuits. U.S. Pat. Publication No. 2007/0255120A1, entitled "Internet-Protocol Based Telemetry Patient Monitoring System ", published on November 1, 2007 to Roznov, describes such a standard patient telemetry system. Roznov, as illustrated in FIGURE 1, describes a system having a plurality of portable telemetry devices 12-18 which are in wireless communication with an access point 20. Portable telemetry devices 12-18 transact IP data packets, bi-directionally, with an information system 22 via a connection 24 of the wired network. The data packets are typically real-time physiological and control information.

Portable telemetry devices 12-18 communicate with devices, e.g., tablet PC 26 on the wired network, e.g., via connection 28, through an access point 20. An optional access point controller 34 controls and directs the signals through access point 20 via a connection 32.

A similar invention is described in International Publication WO 03/025826A2, entitled "Health Monitoring System", published on 27 March 2003 to Gordner et al. There, a patient monitoring system is comprised of patient monitors 2 which are in wireless communication with a centralized application server 1, which stores the patient data for access by multiple workstations 4. The patient monitor could be associated with a defibrillator.

Another similar monitoring invention is described in International Publication WO 2006/071711, entitled "Providing Data Destination Information to a Medical Device", published on 6 July 2006 to Moore et al. Moore et al. teach a medical device which may be configured to transmit medical event information to a selected data destination. Prior to transmission, the user must select and initiate the data transmission in a "push" fashion. The Moore et al. medical device may be an external defibrillator.

An invention which incorporates both a medical device part and a separate communication part within the same housing is described in U.S. Patent Publication 2010/0250697, entitled "Portable Device and Method of Communication Medical Data Information", published on 30 September, 2010 to Hansen et al. The Hansen et al. circuit is intended to separate the complex communications functionality from the critical medical application. The medical device part controls all data flow between it and the communication part.

Another firewall-related invention is described in U.S. Patent Publication 2006/190999A1, entitled "Method and Apparatus for Two-Way Transmission of Medical Data", published on 24 August 2006 to Chen et al. Chen et al. teach a secure firewall located between a healthcare provider and a third party for securely sharing patient image data between the two. The firewall is designed to allow bidirectional data flow with a push-pull control mechanism.

US 2006/030904 discloses a defibrillator monitor telemedicine device with control of access rights.

Each of the afore-described references operates in a similar manner. Each offers a circuit design and associated user interface which is relatively complicated and therefore suboptimal. In particular, the user of each medical monitoring device must take specific action to set-up and/or configure the device to "push" data from the device to the remote station. Each referenced device requires the operator to either set up the monitor defibrillator circuitry to transmit to a chosen location in a chosen format, or must initiate the transmission itself via the user interface. These systems and methods may be appropriate for use in a clinical diagnostic environment, but they are distracting and potentially confusing during an urgent cardiac patient treatment protocol where every moment saved improves the chance for a positive patient outcome. Actions necessary to collect and transmit patient data may divert attention away from the immediate patient care steps, and can introduce a potential for error in either the transmission or the rescue. Third, the recited references each require custom interface software residing both at the device and at the remote station in order to reconcile both sides of the data link and to decode the clinical data from the patient medical device. The interface is expensive to develop and certify for medical use, which must be repeated each time that the interface is modified to meet evolving communications standards. The custom interface software also holds patient data in a customized format which is optimized to the system. Unless the external system contains the same software, the data cannot be read.

Thus what is needed is a method and apparatus for easily sharing patient information obtained by a medical device such as a monitor defibrillator with external systems in a way which avoids the problems presented by the prior art. This object is solved by the independent claims. Advantageous embodiments are defined in the dependent claims.

In accordance with the principles of the present invention, an improved medical device apparatus is described which consists of a medical monitor defibrillator having an integrated telemedicine server. The apparatus includes a patient monitoring circuit and defibrillation therapy circuit which collect patient data and convey it to a clinical processor. A memory receives the patient data from the clinical processor and stores the received patient data in a generic file format. A communications interface is disposed in read-only communication with the memory and transmits the patient data to a remote station. A firewall circuit prevents the writing of any data received from the communications interface to the memory. Each of the circuit elements is contained in a unitary housing.

Also in accordance with the principles of the present invention, a system is described which comprises a monitor defibrillator having an integrated telemedicine server in communications with a transceiver located at a remote station. The system is disposed such that the telemedicine server transmits the patient data to the remote station in response to a request for data from the remote station, i.e. in a "pull" method of data flow. The system thus enables the transmission of data without the need for the operator's action or intervention. By arranging the data stored in the isolated telemedicine server memory via a standard communications interface and in a generic format, the need for customized software to interface with the device and decode the data is eliminated. Lower cost and greater reliability are thus realized.

Also in accordance with the principles of the present invention is an improved method for transferring patient data from a monitor defibrillator telemedicine server having a patient monitoring circuit and a defibrillation therapy circuit to a remote station using a system similar to that described above. The method includes a step of transmitting the patient data from the telemedicine server to the remote station automatically in response to a request from the remote station.

The scope of the invention encompasses medical devices having communications capabilities. In particular, devices such as in-hospital monitor defibrillators used by hospital personnel but separated from a central server and pre-hospital monitor defibrillators used by emergency medical services (EMS) personnel at locations remote from the hospital may benefit from the inventive features.

Another object of the invention is to describe generally a defibrillator monitor which performs non-safety-critical applications and safety-critical applications together, without affecting the safety, reliability and performance of the safety critical functions. Safety critical functions of a defibrillator monitor device include providing critical therapy to a patient (e.g., pacing, CPR and defibrillation therapy) and/or monitoring of a patient (e.g., ECG, blood monitoring such as oxygenation, carbon dioxide and pressure). Non-safety-critical functions and applications include electronic patient care record (ePCR) applications, event summary review and post event analysis. In particular, the invention employs a multi-core processor which runs both functions. A real time operating system (RTOS) and the memory management unit (MMU) run on separate cores of the multi-core processor. Non-safety critical functions run on different cores of the multi-core processor. Thus is established a secure firewalled separation between safety-critical and non-safety critical functions.

### IN THE DRAWINGS:

FIGURE 1 illustrates a medical patient telemetry system according to the prior art.
FIGURE 2 illustrates a block diagram of a defibrillator monitor telemedicine server according to a preferred embodiment of the invention.
FIGURE 3 illustrates one embodiment of a patient data file storage system for use with the defibrillator monitor telemedicine server according to a preferred embodiment of the invention.
FIGURE 4 is an external view of a defibrillator monitor telemedicine server according to an embodiment of the invention.
FIGURE 5 illustrates a block diagram of a defibrillator monitor telemedicine server and remote station system according to another embodiment of the invention.
FIGURE 6 illustrates a flow chart showing a preferred embodiment of the inventive method.
FIGURE 7 illustrates a firewall circuit in which safety-critical and non-safety-critical functions within a device such as a defibrillator monitor telemedicine server are shared within a common multi-core processor, according to one embodiment of the invention.

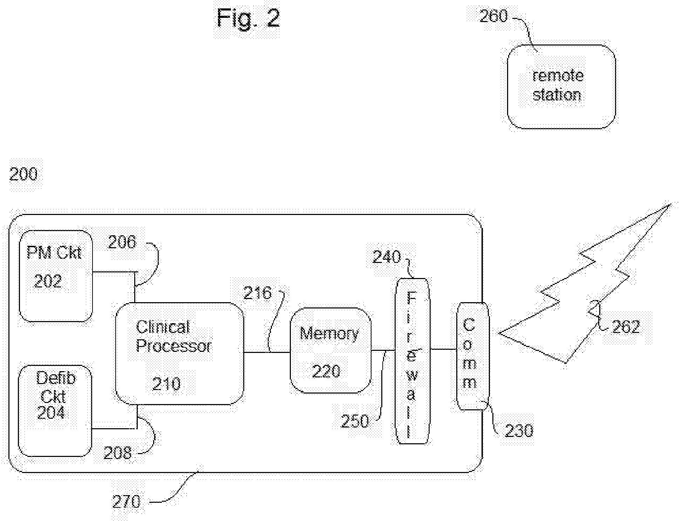

Now turning to the illustrations, FIGURE 2 is a functional block diagram of an exemplary defibrillator monitor telemedicine server 200 of the present invention. Certain functional portions of the defibrillator monitor telemedicine server 200 are defined as safety-critical, which generally consists of hardware and software that relate directly to patient care. Safety-critical features include those circuits used directly for sensing and monitoring a patient's condition. Electrocardiogram (ECG), heart rate, blood pressure and oxygenation, and respiration monitoring are such examples. Safety-critical features also include those circuits used to provide therapy to the patient. Cardiac defibrillation and pacing circuits are such example. Because the failure of a safety-critical circuit can lead to a patient's injury or death, safety-critical circuits must comply with heightened standards of reliability, performance, and security in their design and manufacture.

Defibrillator monitor telemedicine server 200 also includes functional portions that are non-safety critical. Functions such as electronic patient care record applications, event summary review data storage, and post event analysis circuits do not directly affect patient care. Non-safety critical functions generally require a lessened rigor in their design performance and manufacture.

The main elements of the defibrillator monitor telemedicine server 200 shown in FIGURE 2 include a clinical processor 210 which obtains patient and event data from a patient monitoring circuit 202 and a defibrillation therapy circuit 204, an isolable memory 220, a firewall circuit 240, and a communications interface 230 that provides read-only access for external systems to read clinical data stored in memory 220. FIGURE 2 is not intended to depict or preclude any specific hardware or software implementation.

In the FIGURE 2 embodiment, the safety-critical portions of defibrillator monitor telemedicine server 200 include a patient monitoring circuit 202, a defibrillation therapy circuit 204, and a clinical processor circuit 210. The patient monitoring circuit 202 includes sensors which obtain patient medical parameters, generally in real time, and communicate the obtained data to the clinical processor 210 via a patient monitoring data path 206. The defibrillation therapy circuit 204 provides defibrillation and/or pacing therapy to the patient. Defibrillation therapy circuit 204 conveys data regarding sensed patient parameters and defibrillation/pacing operations that are related to the event to clinical processor 210 via defibrillation therapy data path 208.

In a preferred embodiment, clinical processor 210 controls all of the monitoring and therapy functions of DMTS 200. In this embodiment, all functions of clinical processor 210, patient monitoring circuit 201 and defibrillator therapy circuit 204 may be performed by a single processor. The clinical processor 210 may also be operable to capture a record of a rescue event by means of a microphone and/or camera input, not shown in FIGURE 2, to the clinical processor.

In an alternate embodiment, clinical processor 210, patient monitoring circuit 201 and defibrillator therapy circuit 204 are each separate physical processors. In another alternate embodiment, patient monitoring circuit 201 and defibrillator therapy circuit 204 operate independently from clinical processor 210. Clinical processor 210 thus has two primary functions. Clinical processor 210 may be operable to calculate and deliver therapy. Also, clinical processor 210 is operable to collect and process data from the circuits into clinical data, and then to store that processed clinical data for further use.

Clinical processor 210 preferably processes the data from the patient monitoring circuit 201 and defibrillator therapy circuit 204 into processed clinical data that is arranged in generic file formats. The generic file formats are ones that are readable by widely-available software programs. Current examples of such generic file formats include, but are not limited to, extensible mark-up language (.xml), portable document format (.pdf), and/or postscript (.ps). External systems may thus access the clinical data so arranged without requiring any special software.

A less preferred embodiment arranges the clinical data into a custom file format. In this embodiment, any system external to the DMTS 200 will require special software to decode the file information.

Clinical processor 210 saves the processed clinical data to an isolable memory space 220 via a read-write communications path 216. It is noted that clinical processor 210 may require read-access to the clinical data from memory 220 for use with functions, e.g. displays and user indications, internal to the DMTS 200.

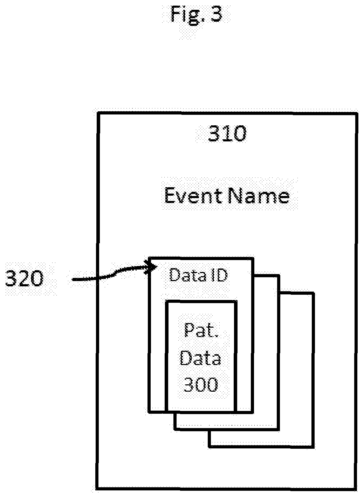

FIGURE 3 illustrates a preferred arrangement for the processed clinical data files as stored in isolable memory 220. The data files that are intended for external system access preferably conform to an established standard for patient clinical data records, such as the National EMS Information System (NEMSIS). As shown in FIGURE 3, patient data records 300 are stored in patient data files 320 with a patient data file name that identifies the nature of the data. The patient data files 320 are in turn arranged in a patient data folder 310, which may be identified by the event name, date and time. The directory of patient data folders 310 in memory 220 is preferably read-only accessible to external systems as long as the DMTS 200 is powered on and active.

Compliance with patient confidentiality rules can be obtained in the above arrangement through a number of methods. Clinical processor 210 may be enabled to anonymize patient data prior to storage in memory 220. Passwords and other appropriate security methods may also be employed to restrict access to the patient data folders 310 by external systems.

Turning back to FIGURE 2, the clinical patient data which resides in isolable memory 220 may be accessed by external systems 260 via a communications interface 230. Preferably, communications interface 230 is a wireless interface of a type which includes but is not limited to Bluetooth.TM, Wi-Fi, or cellular telephone interfaces. Communications interface 230 may also be a wired interface of a type which includes but is not limited to Universal Serial Bus (USB) and Ethernet. The transmission path between the communications interface 230 and the external system 260 is preferably read-only, i.e. transmitted from the communications interface 230 to external system 260 in response to the requesting of patient data from the external system.

A firewall circuit 240 is disposed along a firewalled communications path 250 between isolable memory 220 and communications interface 230. The firewall circuit 240 is operable to allow read-only access at the communications interface 230 to the patient clinical data residing in memory 220. Firewall circuit 240 is further operable to block the writing of any data received from communications interface 230 to memory 220. The firewall circuit 240 thus eliminates any externally-originated avenue for corrupting safety-critical software programs and data. Firewall circuit 240 thus separates the safety critical and non-safety-critical functions of DMTS 200.

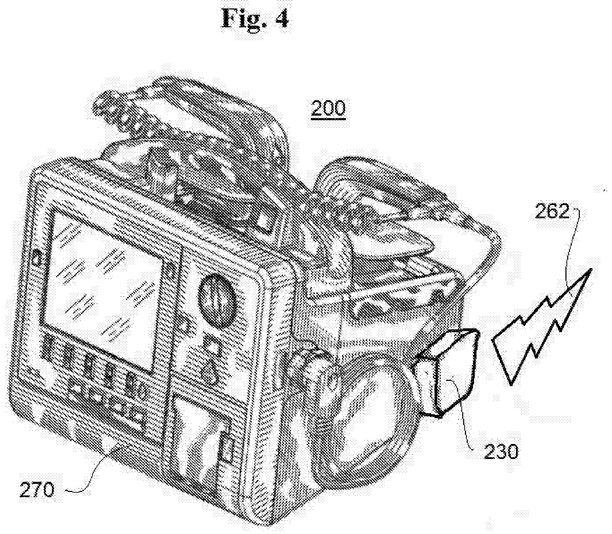

FIGURE 4 illustrates one external view of a DMTS 200. A unitary housing 270 contains each of the patient monitoring circuit 202, the defibrillation therapy circuit 204, the clinical processor 210, the memory 220, and the firewall circuit 240. Shown mounted on the external surface of the housing 270 is the communications interface 230. This is for illustration only, as it is understood that the communications interface circuitry 230 is contained within the housing, and that only the antennae or communications port is disposed externally. Preferably, all components of communications interface 230 are contained inside housing 270. In the illustrated embodiment, the read-only transmission path from the device is a wireless transmission.

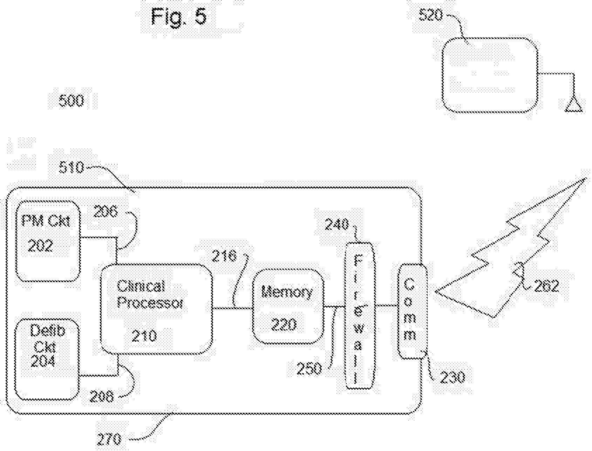

Shown in FIGURE 5 illustrates a block diagram of a defibrillator monitor telemedicine server and remote station system 500 according to another embodiment of the invention. System 500 includes a telemedicine server 510 which is integrated in a common unitary housing with a monitor defibrillator, and a transceiver 520 which is disposed at a central monitoring station at a remote location. The internal arrangement and components of the telemedicine server 510 may be similar to the FIGURE 2 embodiment as previously described. In particular, telemedicine server 510 may include isolable memory 220 protected by a firewall circuit 240. The patient data is stored in the server memory 220. Preferably, the patient data is stored in a generic file format as previously discussed.

Telemedicine server 510 and transceiver 520 are communicatively linked via a read-only transmission path 262. Thus, telemedicine server 510 transmits patient data to the remote station transceiver 520 in response to a request for data from the remote station. The telemedicine server 510 is preferably disposed to require nothing more than the request, without any need for a previous setup or local user action, to perform the data transmission. The communications interface 230 in the telemedicine server 510 and the transceiver 520 may each be wireless or may be wired together.

Telemedicine server 510 is also operable to block, or prevent, external data including that corresponding to the request from the remote station from being written to the internal server memory 220. The firewall circuit 240 prevents the corruption of the safety-critical portions of the system, including for example, the patient monitoring circuit 202 and the defibrillation therapy circuit 204. Circuits 202 and 204 in turn are operable to generate the patient data.

The benefits resulting from the telemedicine server system 500 become clear by description of its operation. By co-locating the non-safety critical communications portions and the safety-critical patient monitoring and therapy portions within the telemedicine server housing 270, the operator has instant telecommunications capability with a remote station as soon as the device is turned on. The server requires no setup prior to operation for transmitting data, because it can obtain an internet identifier automatically from any available network each time it is turned on. At that time, the server can automatically transmit its identifier to a predetermined website fixed within the server memory. Then the server merely awaits an authorized request that initiates the transmission of data stored in memory. The telemedicine server 510 may also be disposed to transmit the patient data to a previously authorized remote transceiver 520 as soon as the patient data is obtained.

The inventive system is also easier to modify as telecommunications protocols change over time. It is well known that safety-critical medical systems require heightened rigor in the design process and are subject to rigorous verification and validation processes in order to be cleared for use. By separating the safety-critical medical systems portions from the telecommunications portions with a firewall 240, the inventors have created a medical device whose communications circuits may be modified with little or no re-validation required of the safety-critical portions, even if all circuits reside in the same unitary housing.

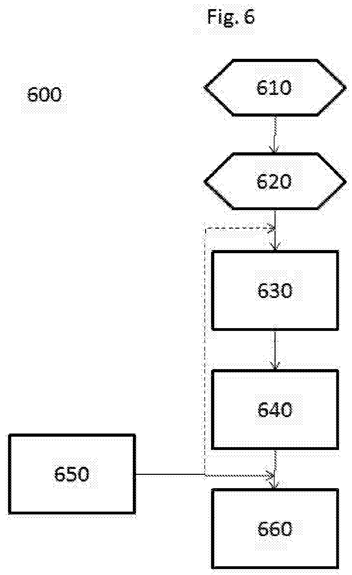

FIGURE 6 illustrates a flow chart showing a method 600 for transferring patient data from a monitor defibrillator to a remote station. The first step 610 includes providing at the patient location a telemedicine server (610) integrated within the housing of the monitor defibrillator. The integrated telemedicine server includes a clinical processor operable to collect patient data from a patient monitoring circuit and a defibrillation therapy circuit. The server also includes a memory operable to receive the patient data from the clinical processor and to store the received patient data in a generic file format. The server interface with external systems includes a communications interface disposed in read-only communication with the memory and further operable to transmit the patient data in the generic file format to the remote station, and a firewall circuit disposed between the memory and the communications interface and operable to prevent the writing of any data received from the communications interface to the memory.

In order to complete the communication setup, a second step 620 includes providing a transceiver at a remote station, and establishing a communications path between it and the communications interface. This second step 620 may be automatically completed when the telemedicine server is turned on in the previous providing step, using communications parameters in the server that are either pre-loaded at the factory, or previously established during prior uses of the server.

After the providing step 610 is completed, the monitor defibrillator is put into use to treat a patient. Patient data is automatically collected by the clinical processor at collecting step 630 and stored in the server memory in the generic file format at storing step 640. Storing step 640 may optionally include storing the patient data in a format which conforms to an established standard for patient clinical data.

The method 600 also contemplates that collecting step 630 and storing step 640 are conducted prior to the establishing of a communications path at step 620. This situation may arise in patient locations in which there is no wired or wireless connectivity available between the server and the remote station.

The remote station transceiver begins the "pull" of patient data from the telemedicine server at requesting step 650. The telemedicine server receives the request via the communications path and automatically determines whether the requester is authorized for data sharing. In addition, the firewall circuit in the server blocks any data from the transceiver to be written in the same memory used to store the patient data.

The upper dashed line in FIGURE 6 from requesting step 650 to the output of the collecting step 630 indicates an optional method in which the telemedicine is turned on and a communications path is established, but wherein a patient is not yet being treated by the device. In this situation, the collecting step 630 may begin after the requesting step 650 is received from the transceiver.

After the determination is made that the remote station is an authorized recipient of data, the telemedicine server transmits the patient data to the remote station via the established communication path at transmitting step 660. Transmitting step 660 preferably occurs automatically and solely in response to the requesting step 650.

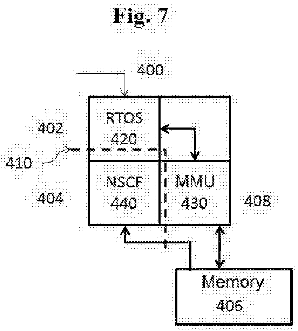

FIGURE 7 illustrates another embodiment of a firewall circuit in which safety-critical and non-safety-critical functions within a device such as a defibrillator monitor telemedicine server are performed by separate processor modules. The separate processor modules are physically separated but reside within a single multi-core processor 400. A firewall circuit 430, which may be a memory management unit, is disposed on one of the separate sub-processors 408. Another of the sub-processors 402 operates a safety-critical clinical processor 420. The clinical processor 420 may run on an RTOS in the sub-processor 402. Yet another of the sub-processors 404 operates a general purpose, non-safety-critical program 440 such as a communications program. The firewall circuit 430 provides read-write capability from the clinical processor 420 to external memory 406. Firewall circuit 430 also blocks any write capability from the non-safety critical program 440 into memory 406. Thus, a secure partition firewall 410 between safety-critical and non-safety critical functions is established.

The above-described embodiment enables a monitor defibrillator device to run safety-critical applications and ePCR applications simultaneously on a single hardware unit. The embodiment may also take advantage of the hardware-assisted virtualization features in a multi-core processor and the secured virtualization support of an RTOS. The embodiment indirectly uses the resource management and protection capabilities of the RTOS and the memory management unit (MMU) of the processor to create a secured partition firewall for running safety-critical applications simultaneously and safely alongside non-safety-critical applications.

The above-described embodiment may be disposed in several ways. One way to safely isolate critical and non-critical functions is to run them on separate processor modules that are physically separated but reside in a single hardware unit or device. Safety-critical applications run on a real time operating system (RTOS) in one of the processors with dedicated CPU, memory and other required devices and peripherals. Non-critical applications run on a general purpose operating system in a separate processor, without the permission of affecting the resources of the safety-critical applications. Display, recording and memory storage unit/module incorporate outputs from both critical and non-critical applications. Safety-critical user interface controls are mapped directly to the safety-critical application in order to be unaffected by reliability, security or performance problems of the non-safety-critical application. Safety-critical applications are thus protected from non-safety-critical applications even though they reside in the same device. Hence the safety-critical applications will function normally even if a non-critical application fails to function correctly.

In an alternate but more efficient and less expensive implementation, the safety-critical applications run directly on an RTOS mapped to one or more dedicated cores of a multi-core processor. The non-safety-critical applications run alongside but on the other cores of the processor mapped to a general purpose operating system, such as Windows.TM, using a secure virtualization technique provided by the RTOS. The multi-core processor chosen for the implementation would support hardware-assisted virtualization features so that the RTOS virtualization implementation can take advantage of the resultant efficiency and improved performance.

Display, recording and storage of data generated from both critical and non-critical applications and the user interface control could also be conducted on separate functional units residing at the patient location. If the display is on a separate unit than where the application is running, then the data and control requests can be transmitted over to/from the display unit over a cable or wirelessly. Regardless of an integrated or separate display, switching between defibrillator/monitor and ePCR applications could be implemented using a single button press or touch of a screen. Defibrillator and monitor functions would be of the highest priority. The device thus retains the capability to automatically switch to defibrillator/monitor functions or alert the user to switch to defibrillator/monitor functions from non-safety related functions if clinical attention is needed.

Modifications to the device, method, and displays as described above are encompassed within the scope of the invention. For example, various configurations of the clinical processer circuits which fulfill the objectives of the described invention fall within the scope of the claims. Also, the particular appearance and arrangement of the external device housing may differ.

It should be understood that, while the present invention has been described in terms of medical applications, the teachings of the present invention are much broader and are applicable for non-medical applications and uses. Further, As one having ordinary skill in the art will appreciate in view of the teachings provided herein, features, elements, components, etc. described in the present disclosure/specification and/or depicted in the appended Figures may be implemented in various combinations of hardware and software, and provide functions which may be combined in a single element or multiple elements. For example, the functions of the various features, elements, components, etc. shown/illustrated/depicted in the Figures can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non volatile storage, etc.) and virtually any means and/or machine (including hardware, software, firmware, combinations thereof, etc.) which is capable of (and/or configurable) to perform and/or control a process.

Moreover, all statements herein reciting principles, aspects, and embodiments of the invention, as well as specific examples thereof, are intended to encompass both structural and functional equivalents thereof. Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Furthermore, exemplary embodiments of the present invention can take the form of a computer program product accessible from a computer-usable and/or computer-readable storage medium providing program code and/or instructions for use by or in connection with, e.g., a computer or any instruction execution system. In accordance with the present disclosure, a computer-usable or computer readable storage medium can be any apparatus that can, e.g., include, store, communicate, propagate or transport the program for use by or in connection with the instruction execution system, apparatus or device. Such exemplary medium can be, e.g., an electronic, magnetic, optical, electromagnetic, infrared or semiconductor system (or apparatus or device) or a propagation medium. Examples of a computer-readable medium include, e.g., a semiconductor or solid state memory, magnetic tape, a removable computer diskette, a random access memory (RAM), a read-only memory (ROM), flash (drive), a rigid magnetic disk and an optical disk. Current examples of optical disks include compact disk - read only memory (CD-ROM), compact disk - read/write (CD-R/W) and DVD. Further, it should be understood that any new computer-readable medium which may hereafter be developed should also be considered as computer-readable medium as may be used or referred to in accordance with exemplary embodiments of the present invention and disclosure.

Having described preferred and exemplary embodiments of systems, devices and methods in accordance with the present invention (which embodiments are intended to be illustrative and not limiting), it is noted that modifications and variations in/to such exemplary embodiments can be made by persons skilled in the art in light of the teachings provided herein (including the appended Figures). It is therefore to be understood that such changes which can be made in/to the preferred and exemplary embodiments of the present disclosure are within the scope of the present invention and the exemplary embodiments disclosed herein.

## Claims

1. A defibrillator monitor telemedicine server (200) comprising:
a patient monitoring circuit (202);
a defibrillation therapy circuit (204);
a clinical processor (210) operable to collect patient data from the patient monitoring circuit and the defibrillation therapy circuit;
an isolable memory (220) operable to receive the patient data from the clinical processor and to store the received patient data in a generic file format;
a communications interface (230) disposed in read-only communication with the isolable memory and further operable to transmit the patient data in the generic file format to a remote station;
a firewall circuit (240) disposed between the isolable memory and the communications interface and operable to prevent the writing of any data received from the communications interface to the isolable memory; and
a unitary housing (270) for containing each of the patient monitoring circuit, the defibrillation therapy circuit, the clinical processor, the isolable memory, the communications interface, and the firewall circuit.

2. The defibrillator monitor telemedicine server of Claim 1, wherein the communications interface is further operable to transmit the patient data unidirectionally to the remote station.

3. The defibrillator monitor telemedicine server of Claim 1, wherein the communications interface comprises a wireless communications interface selected from the group consisting of Bluetooth, WiFi, and cellular telephone.

4. The defibrillator monitor telemedicine server of Claim 1, wherein the communications interface comprises a wired Ethernet interface.

5. The defibrillator monitor telemedicine server of Claim 1, wherein the generic file format is selected from the group consisting of an extensible markup language (XML), portable document format (PDF), and postscript (PS) file format.

6. The defibrillator monitor telemedicine server of Claim 1, wherein the communications interface is further operable to transmit the patient data automatically to the remote station without operator action.

7. The defibrillator monitor telemedicine server of Claim 1, wherein the isolable memory is further operable to store the received patient data in a folder identified by an event title.

8. The defibrillator monitor telemedicine server of Claim 7, wherein the received patient data is stored in the folder arranged by the date/time in which the data was collected.

9. The defibrillator monitor telemedicine server of Claim 1, further comprising a single core processor having a plurality of securely separated sub-processors, wherein the clinical processor and the firewall circuit are operably disposed respectively on one of the plurality of sub-processors.

10. The defibrillator monitor telemedicine server of Claim 9, wherein the clinical processor is further operable to perform safety-critical functions and non-safety-critical functions, and further wherein the safety-critical functions are operably disposed on one of the plurality of sub-processors as a real-time operating system (RTOS).

11. A system for providing patient data from a defibrillator monitor telemedicine server to a remote station, the system comprising:
a defibrillator monitor telemedicine server (510) according to any of Claims 1-10; and
a remote station (260), the remote station comprising a transceiver (520) in communication with the communications interface;
wherein the defibrillator monitor telemedicine server is operable to transmit the patient data to the remote station in response to a request for data from the remote station.

12. The system of Claim 11, wherein the defibrillator monitor telemedicine server is operable to prevent external data received from the remote station from being written to the isolable memory.

13. The system of Claim 11, wherein the patient data is stored in the isolable memory in a generic file format.

14. The system of Claim 11, wherein the defibrillator monitor telemedicine server is further operable to transmit the patient data to the transceiver as the patient data is obtained.

15. A method (600) for transferring patient data from a defibrillator monitor telemedicine server to a remote station, the defibrillator monitor telemedicine server comprising a patient monitoring circuit,
a defibrillation therapy circuit,
a clinical processor operable to collect patient data from the patient monitoring circuit and the defibrillation therapy circuit,
an isolable memory operable to receive the patient data from the clinical processor and to store the received patient data in a generic file format,
a communications interface disposed in read-only communication with the isolable memory and further operable to transmit the patient data in the generic file format to the remote station, and
a firewall circuit disposed between the isolable memory and the communications interface and operable to prevent the writing of any data received from the communications interface to the isolable memory, and
a unitary housing for containing each of the patient monitoring circuit, the defibrillation therapy circuit, the clinical processor, the isolable memory, the communications interface, and the firewall circuit;
wherein the method comprises the steps of:
establishing a communications path (620) between a transceiver at the remote station and the communications interface;
collecting (630) the patient data at the clinical processor;
storing the patient data (640) in the isolable memory in the generic file format;
requesting the patient data (650) from the transceiver to the defibrillator monitor telemedicine server; and
transmitting the patient data (660) from the defibrillator monitor telemedicine server to the remote station automatically in response to the requesting step.

## Patentansprüche

1. Telemedizinischer Server (200) zur Defibrillatorüberwachung, umfassend:
eine Patientenüberwachungsschaltung (202);
eine Defibrillationstherapieschaltung (204);
einen klinischen Prozessor (210), der betriebsfähig ist, um Patientendaten aus der Patientenüberwachungsschaltung und der Defibrillationsüberwachungsschaltung zu sammeln;
einen isolierbaren Speicher (220), der betriebsfähig ist, um die Patientendaten aus dem klinischen Prozessor zu empfangen und die empfangenen Patientendaten in einem generischen Dateiformat zu speichern;
eine Kommunikationsschnittstelle (230), die in Nur-Lese-Kommunikation mit dem isolierbaren Speicher angeordnet ist und ferner betriebsfähig ist, um die Patientendaten in dem generischen Dateiformat an eine entfernte Station zu senden;
eine Firewall-Schaltung (240), die zwischen dem isolierbaren Speicher und der Kommunikationsschnittstelle angeordnet ist und betriebsfähig ist, um zu verhindern, dass Daten, die von der Kommunikationsschnittstelle empfangen werden, in den isolierbaren Speicher geschrieben werden; und
ein einheitliches Gehäuse (270) zum Aufnehmen von jedem von der Patientenüberwachungsschaltung, der Defibrillationstherapieschaltung, dem klinischen Prozessor, dem isolierbaren Speicher, der Kommunikationsschnittstelle und der Firewall-Schaltung.

2. Telemedizinischer Server zur Defibrillatorüberwachung nach Anspruch 1, wobei die Kommunikationsschnittstelle ferner betriebsfähig ist, um die Patientendaten unidirektional an die entfernte Station zu senden.

3. Telemedizinischer Server zur Defibrillatorüberwachung nach Anspruch 1, wobei die Kommunikationsschnittstelle eine drahtlose Kommunikationsschnittstelle ausgewählt aus der Gruppe bestehend aus Bluetooth, WLAN und Mobiltelefon umfasst.

4. Telemedizinischer Server zur Defibrillatorüberwachung nach Anspruch 1, wobei die Kommunikationsschnittstelle eine kabelgebundene Ethernet-Schnittstelle umfasst.

5. Telemedizinischer Server zur Defibrillatorüberwachung nach Anspruch 1, wobei das generische Dateiformat ausgewählt wird aus der Gruppe bestehend aus einer erweiterbaren Auszeichnungssprache (Extensible Markup Language, XML), einem portablen Dokumentenformat (Portable Document Format, PDF) und Postscript-Dateiformat (PS).

6. Telemedizinischer Server zur Defibrillatorüberwachung nach Anspruch 1, wobei die Kommunikationsschnittstelle ferner betriebsfähig ist, um die Patientendaten automatisch ohne Bedieneraktion an die entfernte Station zu senden.

7. Telemedizinischer Server zur Defibrillatorüberwachung nach Anspruch 1, wobei der isolierbare Speicher ferner betriebsfähig ist, um die empfangenen Patientendaten in einem mit einem Ereignistitel bezeichneten Ordner zu speichern.

8. Telemedizinischer Server zur Defibrillatorüberwachung nach Anspruch 7, wobei die empfangenen Patientendaten angeordnet nach dem Datum/der Uhrzeit, an dem bzw. zu der die Daten gesammelt wurden, in dem Ordner gespeichert werden.

9. Telemedizinischer Server zur Defibrillatorüberwachung nach Anspruch 1, ferner umfassend einen Einkernprozessor mit einer Vielzahl von sicher getrennten Subprozessoren, wobei der klinische Prozessor und die Firewall-Schaltung betriebsfähig jeweils auf einem der Vielzahl von Subprozessoren angeordnet sind.

10. Telemedizinischer Server zur Defibrillatorüberwachung nach Anspruch 9, wobei der klinische Prozessor ferner betriebsfähig ist, um sicherheitskritische Funktionen und nicht-sicherheitskritische Funktionen durchzuführen, und wobei ferner die sicherheitskritischen Funktionen betriebsfähig auf einem der Vielzahl von Subprozessoren als ein Echtzeitbetriebssystem (real-time operating system, RTOS) angeordnet sind.

11. System zum Bereitstellen von Patientendaten von einem telemedizinischen Server zur Defibrillatorüberwachung für eine entfernte Station, wobei das System Folgendes umfasst:
einen telemedizinischen Server zur Defibrillatorüberwachung (510) nach einem der Ansprüche 1 bis 10; und
eine entfernte Station (260), wobei die entfernte Station einen Sender-Empfänger (520) in Kommunikationsverbindung mit der Kommunikationsschnittstelle umfasst;
wobei der telemedizinische Server zur Defibrillatorüberwachung betriebsfähig ist, um die Patientendaten als Reaktion auf eine Datenanforderung von der entfernten Station an die entfernte Station zu senden.

12. System nach Anspruch 11, wobei der telemedizinische Server zur Defibrillatorüberwachung betriebsfähig ist, um zu verhindern, dass externe Daten, die von der entfernten Station empfangen werden, in den isolierbaren Speicher geschrieben werden.

13. System nach Anspruch 11, wobei die Patientendaten in einem generischen Dateiformat in dem isolierbaren Speicher gespeichert werden.

14. System nach Anspruch 11, wobei der telemedizinische Server zur Defibrillatorüberwachung ferner betriebsfähig ist, um die Patientendaten an den Sender-Empfänger zu senden, wenn die Patientendaten erhalten werden.

15. Verfahren (600) zum Übertragen von Patientendaten von einem telemedizinischen Server zur Defibrillatorüberwachung an eine entfernte Station, wobei der telemedizinische Server zur Defibrillatorüberwachung Folgendes umfasst:
eine Patientenüberwachungsschaltung,
eine Defibrillationstherapieschaltung,
einen klinischen Prozessor, der betriebsfähig ist, um Patientendaten aus der Patientenüberwachungsschaltung und der Defibrillationsüberwachungsschaltung zu sammeln,
einen isolierbaren Speicher, der betriebsfähig ist, um die Patientendaten aus dem klinischen Prozessor zu empfangen und die empfangenen Patientendaten in einem generischen Dateiformat zu speichern,
eine Kommunikationsschnittstelle, die in Nur-Lese-Kommunikation mit dem isolierbaren Speicher angeordnet ist und ferner betriebsfähig ist, um die Patientendaten in dem generischen Dateiformat an eine entfernte Station zu senden, und
eine Firewall-Schaltung, die zwischen dem isolierbaren Speicher und der Kommunikationsschnittstelle angeordnet ist und betriebsfähig ist, um zu verhindern, dass Daten, die von der Kommunikationsschnittstelle empfangen werden, in den isolierbaren Speicher geschrieben werden, und
ein einheitliches Gehäuse zum Aufnehmen von jedem von der Patientenüberwachungsschaltung, der Defibrillationstherapieschaltung, dem klinischen Prozessor, dem isolierbaren Speicher, der Kommunikationsschnittstelle und der Firewall-Schaltung;
wobei das Verfahren die folgenden Schritte umfasst:
Aufbauen eines Kommunikationspfads (620) zwischen einem Sender-Empfänger an der entfernten Station und der Kommunikationsschnittstelle;
Sammeln (630) der Patientendaten an dem klinischen Prozessor;
Speichern der Patientendaten (640) in dem generischen Dateiformat in dem isolierbaren Speicher;
Anfordern der Patientendaten (650) vom Sender-Empfänger zum telemedizinischen Server zur Defibrillatorüberwachung; und
Senden der Patientendaten (660) von dem telemedizinischen Server zur Defibrillatorüberwachung an die entfernte Station automatisch als Reaktion auf den Anforderungsschritt.

## Revendications

1. Serveur de télémédecine pour moniteur de défibrillateur (200) comprenant :
un circuit de surveillance du patient (202) ;
un circuit de traitement par défibrillation (204) ;
un processeur clinique (210) pouvant être mis en oeuvre pour collecter des données du patient depuis le circuit de surveillance du patient et le circuit de traitement par défibrillation ;
une mémoire isolable (220) pouvant être mise en oeuvre pour recevoir les données du patient provenant du processeur clinique et pour stocker les données du patient reçues dans un format de fichier générique ;
une interface de communications (230) disposée en communication en lecture seule avec la mémoire isolable et pouvant être mise en oeuvre en outre de manière à transmettre les données du patient dans le format de fichier générique à un poste distant ;
un circuit pare-feu (240) disposé entre la mémoire isolable et l'interface de communications et pouvant être mis en oeuvre pour empêcher l'inscription d'éventuelles données provenant de l'interface de communications dans la mémoire isolable ; et
un logement monobloc (270) pour contenir chacun parmi le circuit de surveillance du patient, le circuit de traitement par défibrillation, le processeur clinique, la mémoire isolable, l'interface de communications et le circuit pare-feu.

2. Serveur de télémédecine pour moniteur de défibrillateur selon la revendication 1, dans lequel l'interface de communications peut en outre être mise en oeuvre pour transmettre les données du patient de manière unidirectionnelle au poste distant.

3. Serveur de télémédecine pour moniteur de défibrillateur selon la revendication 1, dans lequel l'interface de communications comprend une interface de communications sans fil sélectionnée à partir du groupe constitué par le Bluetooth, le WiFi et un téléphone mobile.

4. Serveur de télémédecine pour moniteur de défibrillateur selon la revendication 1, dans lequel l'interface de communications comprend une interface Ethernet filaire.

5. Serveur de télémédecine pour moniteur de défibrillateur selon la revendication 1, dans lequel le format de fichier générique est sélectionné à partir du groupe constitué par un langage de balisage extensible (XML), un format de document portable (PDF) et un format de fichier postscript (PS).

6. Serveur de télémédecine pour moniteur de défibrillateur selon la revendication 1, dans lequel l'interface de communications peut en outre être mise en oeuvre pour transmettre les données du patient automatiquement au poste distant sans action de l'opérateur.

7. Serveur de télémédecine pour moniteur de défibrillateur selon la revendication 1, dans lequel la mémoire isolable peut en outre être mise en oeuvre pour stocker les données du patient reçues dans un dossier identifié par un titre d'événement.

8. Serveur de télémédecine pour moniteur de défibrillateur selon la revendication 7, dans lequel les données du patient reçues sont stockées dans le dossier agencé selon la date/l'heure à laquelle les données ont été collectées.

9. Serveur de télémédecine pour moniteur de défibrillateur selon la revendication 1, comprenant en outre un processeur simple coeur ayant une pluralité de sous-processeurs séparés en toute sécurité, dans lequel le processeur clinique et le circuit pare-feu sont disposés de manière opérationnelle respectivement sur l'un de la pluralité des sous-processeurs.

10. Serveur de télémédecine pour moniteur de défibrillateur selon la revendication 9, dans lequel le processeur clinique peut en outre être mis en oeuvre pour réaliser des fonctions critiques en matière de sécurité et des fonctions non critiques en matière de sécurité, et en outre dans lequel les fonctions critiques en matière de sécurité sont disposées de manière opérationnelle sur l'un de la pluralité des sous-processeurs comme un système d'exploitation en temps réel (RTOS).

11. Système pour fournir des données de patient provenant d'un serveur de télémédecine pour moniteur de défibrillateur à un poste distant, le système comprenant :
un serveur de télémédecine pour moniteur de défibrillateur (510) selon l'une quelconque des revendications 1 à 10 ; et
un poste distant (260), le poste distant comprenant un émetteur-récepteur (520) en communication avec l'interface de communications ;
dans lequel le serveur de télémédecine pour moniteur de défibrillateur peut être mis en oeuvre pour transmettre les données du patient au poste distant en réponse à une demande de données provenant du poste distant.

12. Système selon la revendication 11, dans lequel le serveur de télémédecine pour moniteur de défibrillateur peut être mis en oeuvre pour empêcher que les données externes reçues du poste distant ne soient inscrites sur la mémoire isolable.

13. Système selon la revendication 11, dans lequel les données du patient sont stockées dans la mémoire isolable dans un format de fichier générique.

14. Système selon la revendication 11, dans lequel le serveur de télémédecine pour moniteur de défibrillateur peut en outre être mis en oeuvre pour transmettre les données du patient à l'émetteur-transmetteur lorsque les données du patient sont obtenues.

15. Procédé (600) pour transférer des données d'un patient depuis un serveur de télémédecine pour moniteur de défibrillateur à un poste distant, le serveur de télémédecine pour moniteur de défibrillateur comprenant un circuit de surveillance du patient,
un circuit de traitement par défibrillation,
un processeur clinique pouvant être mis en oeuvre pour collecter les données du patient depuis le circuit de surveillance du patient et le circuit de traitement par défibrillation,
une mémoire isolable pouvant être mise en oeuvre pour recevoir les données du patient provenant du processeur clinique et pour stocker les données du patient reçues dans un format de fichier générique,
une interface de communications disposée en communication en lecture seule avec la mémoire isolable et pouvant en outre être mise en oeuvre pour transmettre les données du patient dans un format de fichier générique au poste distant, et
un circuit pare-feu disposé entre la mémoire isolable et l'interface de communications et pouvant être mis en oeuvre pour empêcher l'inscription d'éventuelles données reçues de l'interface de communications sur la mémoire isolable, et
un logement monobloc pour contenir chacun parmi le circuit de surveillance du patient, le circuit de traitement par défibrillation, le processeur clinique, la mémoire isolable, l'interface de communications et le circuit pare-feu ;
dans lequel le procédé comprend les étapes consistant à :
établir un chemin de communications (620) entre un émetteur-récepteur au poste distant et l'interface de communication ;
collecter (630) les données du patient sur le processeur clinique ;
stocker les données du patient (640) dans la mémoire isolable dans le format de fichier générique ;
demander les données du patient (650) provenant de l'émetteur-récepteur au serveur de télémédecine de moniteur de défibrillateur ; et
transmettre les données du patient (660) du serveur de télémédecine pour moniteur de défibrillateur au poste distant automatiquement en réponse à l'étape de demande.
